# EUROPÄISCHE PATENTSCHRIFT

(11) **EP 1 082 074 B1**
(45) Veröffentlichungstag und Bekanntmachung des Hinweises auf die Patenterteilung: **28.12.2005**
(21) Anmeldenummer: 98921323.6
(22) Anmeldetag: 03.06.1998
(51) Int. Cl.: A61F 2/30, A61F 2/34

(54) **IMPLANTAT AUS KUNSTSTOFF MIT METALLNETZ**
PLASTIC IMPLANT WITH METAL NETTING
IMPLANT EN PLASTIQUE DOTE D'UN RESEAU METALLIQUE

(43) Veröffentlichungstag der Anmeldung: 14.03.2001
(73) Patentinhaber: Mathys Medizinaltechnik AG, 2544 Bettlach (CH)
(72) Erfinder: STAUDTE, Hans-Walter, D-52146 Wrüselen (DE); BAMERT, Peter, CH-4500 Solothurn (CH); STUDER, Peter, CH-4552 Derendingen (CH)
(74) Vertreter: Lusuardi, Werther Giovanni
(86) Internationale Anmeldenummer: PCT/CH1998/000234
(87) Internationale Veröffentlichungsnummer: WO 1999/062438

(56) Entgegenhaltungen:
- EP-A- 0 149 975
- EP-A- 0 190 422
- EP-A- 0 212 087
- EP-A- 0 330 606
- EP-A- 0 338 976
- EP-A- 0 341 199
- EP-A- 0 677 297
- WO-A-90/08520
- WO-A-93/07835
- DE-C- 3 901 811
- FR-A- 2 539 209
- FR-A- 2 620 623
- GB-A- 2 059 267
- US-A- 4 195 368
- US-A- 4 846 834

## Beschreibung

Die Erfindung betrifft ein Implantat gemäss dem Patentanspruch 1.

Das Implantat gemäß EP-A-190 422 weist die Merkmale gemäß dem Oberbegriff des Patentanspruchs 1 auf.

Aus der EP-B 212087 ist eine künstliche Hüftpfanne bekannt, welche aus einer halbkugelförmigen Kunststoffschale besteht, auf deren, zur Anlage an den Beckenknochen bestimmten Aussenseite ein mehrlagiges metallisches Netz aufgebracht ist. Durch diese Anordnung ist ein Knochenwachstum in die offenen Maschen des Netzes möglich.
Die Nachteile bei dieser bekannten Hüftpfanne sind die folgenden:
a) das Vorhandensein von freien Oberflächenregionen der aus Kunststoff bestehenden Schalenaussenseite, welche nicht vom Netz überdeckt sind, hindern das Anwachsen des Knochens;
b) die Mehrlagigkeit des Netzes, welche die freien Oberflächenregionen etwas schwerer zugänglich macht, bewirkt eine erhebliche (ungünstige) Veränderung der Steifigkeit der Hüftpfanne; und
c) im Revisionsfall wird durch den knöchernen Einbau das Pfannenlager unter Umständen stark beschädigt.

Hier will die Erfindung Abhilfe schaffen. Der Erfindung liegt das Problem zugrunde, ein Implantat zu schaffen, welches unter minimaler Beeinflussung seiner Steifigkeit ein optimales osseointegratives Verhalten zeigt.

Die Erfindung löst die gestellte Aufgabe mit einem chirurgischen Implantat, welches die Merkmale des Anspruchs 1 aufweist.

Damit ist der Vorteil erzielbar, dass die gesamte Oberfläche des Implantates - welche nun von einem dichten Metallnetz geschützt ist- ein optimales Anwachsen des Knochens - statt der üblichen Verankerung des Knochens im Metallnetz - gestattet. Durch die Struktur des Tressengewebes des Metallnetzes ergibt sich ein optimales Anwachsverhalten des Knochens an das Implantat.

Unter dem Begriff "Tressengewebe" wird ein Gewebe mit Leinenbindung verstanden, bei welchem jedoch die Kettfäden weit auseinander und die Schussfäden dicht aneinander liegen. Im weiteren sind die Kettfäden stärker als die Schussfäden. Die "Leinenbindung" ist auch als "glatte Bindung" bekannt. Ein Kettfaden bindet sich jeweils über einen Schussfaden und umgekehrt.

Beim erfindungsgemäss verwendeten "Tressengewebe" sind die Kettdrähte in einem solchen Abstand zueinander angeordnet, dass der Schussdraht gerade noch um den Kettfaden gebogen werden kann, ohne dass er reisst. Zum Zwecke bestmöglicher Anwachsung des Knochens haben sich insbesondere Drähte aus Titan oder Legierungen als vorteilhaft erwiesen. Grundsätzlich wären auch andere Metalllegierungen verwendbar, welche ähnliche osteokonduktive Eigenschaften wie Titan aufweisen.

Die Elastizität des Metallnetzes beeinflusst die Steifigkeit des Kunststoffkerns des Implantats nicht stärker. Durch die relativ grosse Dicke des erfindungsgemässen Metallnetzes, von typischerweise 1,1 bis 2,2 mm, vorzugsweise von 1,5 bis 2,0 mm, ist es möglich, ein einlagiges Netz zu verwenden und bei typischerweise 20 % - 80 %, vorzugsweise 30 % - 60 % Einpresstiefe des Metallnetzes in die Oberfläche des Kunststoffes (z.B. Polyäthylen) einen genügenden Sicherheitsabstand zum Knochen zu erreichen.

Die Kettdrähte des Tressengewebes weisen einen grösseren Abstand zueinander auf als die Schussdrähte. Der Abstand zwischen den Kettdrähten beträgt typischerweise 1,0 bis 3,0 mm, vorzugsweise 1,3 bis 1,8 mm; derjenige zwischen den Schussdrähten ist typischerweise kleiner als 0,4 mm, vorzugsweise kleiner als 0,2 mm.

Der Durchmesser der Kettdrähte des Tressengewebes ist grösser als der Durchmesser der Schussdrähte. Der Durchmesser der Kettdrähte liegt typischerweise zwischen 0,6 und 1,2 mm, vorzugsweise zwischen 0,8 und 1,0 mm; derjenige der Schussdrähte zwischen 0,3 und 0,6 mm, vorzugsweise zwischen 0,45 und 0,55 mm. Damit ergibt sich eine vorteilhafte Makrostruktur des erfindungsgemässen Implantats.

Die Osseointegration kann noch zusätzlich verbessert werden durch Aufrauhung der Drahtoberfläche des Metallnetzes, z.B. durch Beizen. Die Oberflächenrauhigkeit der Kettdrähte und Schussdrähte liegt typischerweise zwischen 5 und 100 µm, und vorzugsweise zwischen 10 und 70 µm. Diese Mikrostruktur fördert das Anwachsen des Knochens an das Implantat.

Bei einer weiteren bevorzugten Ausführung weist der Kunststoffkern mindestens zwei durchgehende Löcher zur Aufnahme von Knochenschrauben auf (Erzeugung einer guten Primärstabilität) sowie Sacklöcher zur Aufnahme eines Handhabungsinstrumentes.

Der Kunststoffkern des chirurgischen Implantats kann auch eine Verstärkung aufweisen, vorzugsweise in Form von metallischen Armierungen oder nichtmetallischen Fasern.

Das erfindungsgemässe Implantat kann für eine Vielzahl von Anwendungen eingesetzt werden. Im folgenden wird es jedoch allein im Zusammenhang mit einer Endogelenkprothese, insbesondere einer künstliche Hüftpfanne näher beschrieben, welche vorzugsweise zwei radial von der Aussenfläche des Kunststoffkern abstehende Zapfen aufweist, welche primär als Rotationssicherung dienen.

Es zeigen:
Fig. 1 eine schematische Darstellung einer erfindungsgemässen künstlichen Hüftpfanne in der Seitenansicht;
Fig. 2 eine um 90° verdrehte schematische Darstellung der Hüftpfanne nach Fig. 1;
Fig. 3 eine Aufsicht auf ein Metallnetz für die erfindungsgemässe Hüftpfanne;
Fig. 4 einen Längsschnitt durch einen Kettdraht des Metallnetzes gemäss Fig. 3 senkrecht zur Netzebene; und
Fig. 5 eine Vorderansicht auf den Querschnitt durch das Metallnetzes gemäss Fig. 3 senkrecht zur Netzebene.

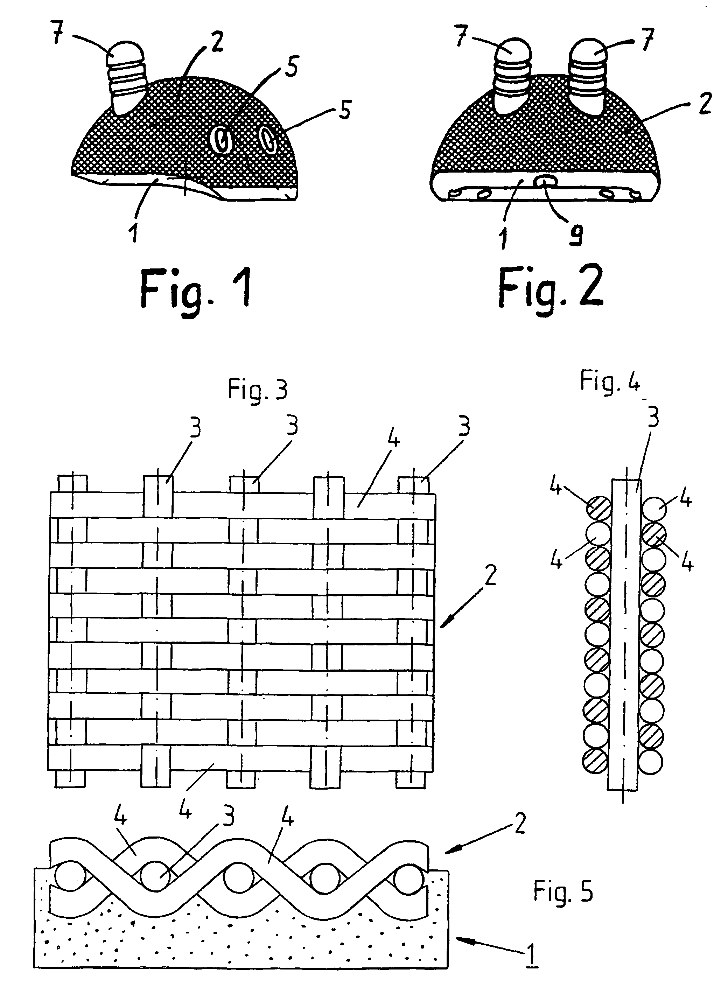

Die in den Fig. 1 und 2 dargestellte künstliche Hüftpfanne besteht im wesentlichen aus einem kugelschalenförmigen Kunststoffkern 1 (z.B. aus UHMW PE; Ultra High Molecular Weight Polyethylen), auf dessen Aussenfläche - die zur Anlage an den Beckenknochen bestimmt ist - ein Metallnetz 2 aus Reintitan aufgebracht ist.
Wie in den Fig. 3 - 5 im Detail dargestellt sind die Kettdrähte 3 und Schussdrähte 4 des Metallnetzes 2 als Leinenbindung zu einem Tressengewebe verwoben.
Die künstliche Hüftpfanne weist ferner vier durchgehende Löcher 5 zur Aufnahme von Knochenschrauben sowie zwei radial von der Aussenfläche des Kunststoffkerns 1 abstehende Zapfen 7 auf. Die Löcher 5 gestatten eine Verschraubung, d.h. zusätzliche Verankerung der Hüftpfanne im Beckenknochen und die Zapfen 7 dienen zur Rotationssicherung.
Im weiteren sind an der Peripherie der künstliche Hüftpfanne Sacklöcher 9 angebracht, in welche ein Handhabungsinstrument einführbar ist, um die Hüftpfanne handhaben und positionieren zu können.

Die den Femurkopf aufnehmende Innenfläche der künstlichen Hüftpfanne ist naturgemäss weder armiert noch beschichtet, um die tribologischen Eigenschaften nicht zu verschlechtern. Das Metallnetz 2 wird somit nur auf denjenigen Oberflächen des Kunststoffkerns 1 aufgetragen, an welchen ein Anwachsen des Knochens erfolgen soll.

Wie in den Fig. 3 - 5 dargestellt, weist das Metallnetz 2 ein Tressengewebe auf. Die Kettdrähte 3 des Metallnetzes 2 weisen einen Abstand von 1,3 bis 1,8 mm zueinander auf, währenddem die Schussdrähte 4 dicht aneinander liegen, so dass praktisch kein Abstand dazwischen liegt. Der Durchmesser der Kettdrähte 3 beträgt 0,8 bis 1,0 mm und ist grösser als der Durchmesser der Schussdrähte 4, welcher 0,45 bis 0,55 mm beträgt.
Die Oberflächenrauhigkeit der Kettdrähte 3 und Schussdrähte 4 liegt bei 10 bis 70 *µ*m und wird durch Beizen des Metallnetzes 2 erzielt.
Das 1,5 bis 2,0 mm dicke Metallnetz 2 wird - wie in Fig. 5 angedeutet - in einem ersten fabrikatorischen Schritt zu 30 % bis 60 % seiner Dicke in den Kunststoffkern 1 eingebettet, so dass ein fester mechanischer Verbund zwischen Kunststoffkern 1 und Metallnetz 2 resultiert.

## Patentansprüche

1. Implantat aus einem Kunststoffkern (1), welcher an seiner Oberfläche durch ein einlagiges Metallnetz (2) überdeckt ist,
**dadurch gekennzeichnet, dass**
A) das Metallnetz aus Kettdrähten (3) und Schussdrähten (4) mittels Leinenbindung zu einem Tressengewebe verwoben ist, dessen Schussdrähte (4) dicht aneinander liegen; und
B) der freie Abstand zwischen den Schussdrähten (4) kleiner als 0,2 mm ist.

2. Implantat nach Anspruch 1, **dadurch gekennzeichnet, dass** die Kettdrähte (3) einen grösseren Abstand zueinander aufweisen als die Schussdrähte (4).

3. Implantat nach Anspruch 1 oder 2, **dadurch gekennzeichnet, dass** der freie Abstand zwischen den Kettdrähten (3) 1,0 bis 3,0 mm beträgt.

4. Implantat nach Anspruch 3, **dadurch gekennzeichnet, dass** der freie Abstand zwischen den Kettdrähten (3) 1,3 bis 1,8 mm beträgt.

5. Implantat nach einem der Ansprüche 1 bis 4, **dadurch gekennzeichnet, dass** der Durchmesser der Kettdrähte (3) grösser ist als der Durchmesser der Schussdrähte (4).

6. Implantat nach einem der Ansprüche 1 bis 5, **dadurch gekennzeichnet, dass** der Durchmesser der Kettdrähten (3) zwischen 0,6 und 1,2 mm, vorzugsweise zwischen 0,8 und 1,0 mm liegt.

7. Implantat nach einem der Ansprüche 1 bis 6, **dadurch gekennzeichnet, dass** der Durchmesser der Schussdrähte (4) zwischen 0,3 und 0,6 mm, vorzugsweise zwischen 0,45 und 0,55 mm liegt.

8. Implantat nach einem der Ansprüche 1 bis 7, **dadurch gekennzeichnet, dass** die Oberflächenrauhigkeit der Kettdrähte (3) und Schussdrähte (4) zwischen 5 und 100 µm, vorzugsweise zwischen 10 und 70 µm liegt.

9. Implantat nach einem der Ansprüche 1 bis 8, **dadurch gekennzeichnet, dass** die Dicke des Metallnetzes zwischen 1,1 und 2,2 mm, vorzugsweise zwischen 1,5 und 2,0 mm liegt.

10. Implantat nach einem der Ansprüche 1 bis 9, **dadurch gekennzeichnet, dass** das Metallnetz (2) zu 20 - 80 %, vorzugsweise zu 30 - 60 % seiner Dicke im Kunststoff (1) eingebettet ist.

11. Implantat nach einem der Ansprüche 1 bis 10, **dadurch gekennzeichnet, dass** das Metallnetz (2) aus Titan oder Legierungen davon besteht.

12. Implantat nach einem der Ansprüche 1 bis 11, **dadurch gekennzeichnet, dass** der Kunststoffkern (1) mindestens zwei durchgehende Löcher (5) zur Aufnahme von Knochenschrauben aufweist.

13. Implantat nach einem der Ansprüche 1 bis 12, **dadurch gekennzeichnet, dass** der Kunststoffkern (1) Sacklöcher (9) zur Aufnahme eines Handhabungsinstrumentes aufweist.

14. Implantat nach einem der Ansprüche 1 bis 13, **dadurch gekennzeichnet, dass** es eine Endogelenkprothese ist, insbesondere eine künstliche Hüftpfanne, welche vorzugsweise zwei radial von der Aussenfläche des Kunststoffkern (1) abstehende Zapfen (7) aufweist.

## Claims

1. An implant made of a plastic core (1) that is covered on its surface by a single-layer metal mesh (2),
**characterised in that**
A) the metal mesh is made of warp wires (3) and weft wires (4) interwoven in plain weave so as to form a knitted fabric, the weft wires (4) of which are arranged close to one another; and
B) the clear space between the weft wires (4) is smaller than 0.2 mm.

2. An implant as claimed in claim 1, **characterised in that** the warp wires (3) are spaced at wider intervals than the weft wires (4).

3. An implant as claimed in claim 1 or 2, **characterised in that** the clear space between the warp wires (3) is between 1.0 mm and 3.0 mm.

4. An implant as claimed in claim 3, **characterised in that** the clear space between the warp wires (3) is between 1.3 mm and 1.8 mm.

5. An implant as claimed in one of the claims 1 to 4, **characterised in that** the diameter of the warp wires (3) is greater than the diameter of the weft wires (4).

6. An implant as claimed in one of the claims 1 to 5, **characterised in that** the diameter of the warp wires (3) is between 0.6 and 1.2 mm, preferably between 0.8 and 1.0 mm.

7. An implant as claimed in one of the claims 1 to 6, **characterised in that** the diameter of the weft wires (4) is between 0.3 and 0.6 mm, preferably between 0.45 and 0.55 mm.

8. An implant as claimed in one of the claims 1 to 7, **characterised in that** the surface roughness of the warp wires (3) and the weft wires (4) is between 5 and 100 µm, preferably between 10 and 70 µm.

9. An implant as claimed in one of the claims 1 to 8, **characterised in that** the thickness of the metal mesh is between 1.1 and 2.2 mm, preferably between 1.5 mm and 2.0 mm.

10. An implant as claimed in one of the claims 1 to 9, **characterised in that** the metal mesh (2) is embedded, on 20 to 80 %, preferably on 30 to 60 % of its thickness, in the plastic material (1).

11. An implant as claimed in one of the claims 1 to 10, **characterised in that** the metal mesh (2) is made of titanium or alloys thereof.

12. An implant as claimed in one of the claims 1 to 11, **characterised in that** the plastic core (1) has at least two through holes (5) for receiving bone screws.

13. An implant as claimed in one of the claims 1 to 12, **characterised in that** the plastic core (1) is provided with blind holes (9) for receiving a handling instrument.

14. An implant as claimed in one of the claims 1 to 13, **characterised in that** it is a joint endoprosthesis, particularly an artificial acetabulum that is preferably provided with two pins (7) radially protruding from the outer surface of the plastic core (1).

## Revendications

1. Implant composé d'un noyau (1) en matière plastique, lequel est recouvert d'un filet métallique à couche unique (2),
**caractérisé en ce que**
A) le filet métallique est tissé à partir de fils de chaîne (3) et de fils de trame (4) au moyen d'une armure toile afin d'obtenir un tissu tressé dont les fils de trame (4) sont disposés très près les uns des autres; et
B) l'espace libre entre les fils de trame (4) est inférieur à 0,2 mm.

2. Implant selon la revendication 1, **caractérisé en ce que** les fils de chaîne (3) présentent entre eux un espace plus important que les fils de trame (4).

3. Implant selon la revendication 1 ou 2, **caractérisé en ce que** l'espace libre entre les fils de chaîne (3) est compris entre 1,0 et 3,0 mm.

4. Implant selon la revendication 3, **caractérisé en ce que** l'espace libre entre les fils de chaîne (3) est compis entre 1,3 et 1,8 mm.

5. Implant selon l'une des revendications 1 à 4, **caractérisé en ce que** le diamètre des fils de chaîne (3) est supérieur au diamètre des fils de trame (4).

6. Implant selon l'une des revendications 1 à 5, **caractérisé en ce que** le diamètre des fils de chaîne (3) est compris entre 0,6 et 1,2 mm, de préférence entre 0,8 et 1,0 mm.

7. Implant selon l'une des revendications 1 à 6, **caractérisé en ce que** le diamètre des fils de trame (4) est compris entre 0,3 et 0,6 mm, de préférence entre 0,45 et 0,55 mm.

8. Implant selon l'une des revendications 1 à 7, **caractérisé en ce que** la rugosité de surface des fils de chaîne (3) et des fils de trame (4) est de 5 à 100 µm, de préférence de 10 à 70 µm.

9. Implant selon l'une des revendications 1 à 8, **caractérisé en ce que** l'épaisseur du filet métallique est de 1,1 à 2,2 mm, de préférence de 1,5 à 2,0 mm.

10. Implant selon l'une des revendications 1 à 9, **caractérisé en ce que** le filet métallique (2) est encastré dans la matière plastique (1) à 20 - 80 %, de préférence à 30 - 60 %, de son épaisseur.

11. Implant selon l'une des revendications 1 à 10, **caractérisé en ce que** le filet métallique (2) est en titane ou en alliages de titane.

12. Implant selon l'une des revendications 1 à 11, **caractérisé en ce que** le noyau (1) en matière plastique présente au moins deux trous traversants (5) destinés à recevoir des vis d'ostéosynthèse.

13. Implant selon l'une des revendications 1 à 12, **caractérisé en ce que** le noyau (1) en matière plastique présente des trous borgnes (9) destinés à recevoir un instrument de manipulation.

14. Implant selon l'une des revendications 1 à 13, **caractérisé en ce que** ledit implant est une endoprothèse articulaire, notamment une cavité artificielle de la hanche, laquelle présente deux tenons dépassant radialement de la surface extérieure du noyau (1) en matière plastique.
